# EUROPÄISCHE PATENTSCHRIFT

(11) **EP 0 493 732 B1**
(45) Veröffentlichungstag und Bekanntmachung des Hinweises auf die Patenterteilung: **17.04.1996**
(21) Anmeldenummer: 91121471.6
(22) Anmeldetag: 14.12.1991
(51) Int. Cl.: C12S 13/00

(54) **Verfahren zur Aufarbeitung von Altgummi**
Process for processing of old rubber
Procédé pour le traitement de vieux caoutchouc

(30) Priorität: 22.12.1990 DE 4042009
(43) Veröffentlichungstag der Anmeldung: 08.07.1992
(73) Patentinhaber: HÖLZEMANN METALLVERARBEITUNG GmbH, D-86641 Rain (DE)
(72) Erfinder: Straube, Gunhild, O-4090 Halle-Neustadt (DE); Straube, Eckhardt, O-4090 Halle-Neustadt (DE); Neumann, Willi, Prof.Dr., O-4090 Halle-Neustadt (DE); Rückauf, Helmut, Dr., O-4090 Halle-Neustadt (DE); Forkmann, Ralf, Dipl.-Ing., O-4090 Halle-Neustadt (DE); Löffler, Martin, O-1160 Berlin (DE)
(74) Vertreter: Böhme, Volker, Dipl.-Ing.

(56) Entgegenhaltungen:
- EP-A- 0 402 704
- GB-A- 2 097 817
- APPLIED BIOCHEMISTRY & BIOTECHNOLOGY, Bände 24-25, Frühling-Sommer 1990; D. RAGHAVAN et al., Seiten 387-396
- PROCEEDINGS OF THE 11th SYMPOSIUM ON BIO-TECHNOLOGY FOR FUELS & CHEMICALS, 08-12 Mai 1989, Colorado, Springs, CO (US)

## Beschreibung

Die Erfindung betrifft ein Verfahren zur Aufarbeitung von Altgummi, bei dem der Altgummi Schwefel enthält und zerkleinert wird und bei dem aus dem zerkleinerten Altgummi unter Devulkanisierung Kautschukregenerat erzeugt wird.

Jährlich fallen weltweit mehrere Millionen Tonnen Altgummi, insbesondere alte Autoreifen an, die bisher nur unzureichend als Sekundärrohstoff genutzt werden. Aufgrund des geringen Anteils des durch verschiedene Aufarbeitungsvarianten, z.B. Runderneuerung von Reifen, in den Stoffkreislauf zurückgeführten Gummis kommt es zu einer mengenmäßigen Anhäufung von Altreifen auf Deponien, die einer Aufarbeitung bedürfen. Bisher wird davon ausgegangen, daß es sich bei Altreifen um natürlich unzersetzliche Abfallstoffe handelt (DE 2638387). Die bisherige Aufarbeitung der Altreifen erfolgt auf drei verschiedenen Verfahrenswegen.

Als erstes werden die Altreifen nach einer Granulierung bis zu Korngrößen von etwa 25 mm als Zusatz zur Herstellung von bituminösen Straßendeckschichten oder Sportplatzbefestigungen verwendet. Dazu wird das Gummigranulat in den im Straßenbau üblichen Mischern mit Zement, Kiessand oder Sand, Wasser und Haftverbesserern gemischt und mit den üblichen Straßenbaumaschinen als hochelastische Zwischenschicht, beispielsweise bei der Autobahnrekonstruktion oder beim Straßengleisbau, verwendet (DE 2638387).

Eine zweite Möglichkeit, Altreifen als Sekundärrohstoff zu verwerten, besteht darin, die Altreifen einer Pyrolyse zu unterziehen, um dadurch Pyrolyseöl als Ausgangsstoff für chemische Grundstoffe oder Brennöl zur Gewinnung von Wärmeenergie und Elektroenergie durch Ausnutzung der thermischen Energie des Schwelgases durch direkten Antrieb einer Gasturbine zu gewinnen (DE 2724813). Dazu werden die Altreifen unterkühlt. Die unterkühlten Reifen werden in der Regel zunächst einem Gummivorbrecher zugeführt, in dem nur der Gummi der Reifen derart vorgebrochen wird, daß er von den Wulstdrähten gelöst werden kann. Dem Gummivorbrecher wird z.B. eine Doppelrotorhammermühle nachgeschaltet, die die Grundbestandteile von der Karkasse abschlägt, die danach in einer Siebtrommel in verschiedene Korngrößen sortiert werden. Das Gummigranulat wird dann über Magnetabscheider und Siebanlagen von den restlichen Metallanteilen und Cordfasern getrennt (DE 2724813). Anschließend werden die Gummibestandteile bei etwa 500^{o}C entgast. Der dabei entstehende Ruß kann entweder als Weiterverarbeitungsprodukt oder als Brennstoff verwendet werden. Das durch die Entgasung entstehende Schwelgas wird zum direkten Antrieb einer Gasturbine und somit zur Energieerzeugung genutzt.

Eine dritte Möglichkeit, Altreifen als Sekundärrohstoff zu verwenden, besteht darin, feinzerkleinertes Gummigranulat, z.B. das bei der Runderneuerung von Altreifen anfallende Gummimehl, vorzugsweise im Extruder zu replastizieren und bis zu 20 Gewichtsanteilen an der Protektormischung bei der Runderneuerung von Altreifen wiederzuverwenden. Offensichtliche Nachteile dieses Verfahrens sind die hohen Anlagen- und Energiekosten des Extruders bzw. analoger Einrichtungen sowie der Einsatz von Chemikalien, die einen Ketten- und/oder Vernetzerabbau bewirken und die ganz oder teilweise im Replastikat verbleiben. Zudem kann ein nur relativ geringer Anteil der anfallenden Altreifenmenge durch Regenerierung einer Wiederverwertung zugeführt werden.

Eine Aufgabe der Erfindung ist es, ein Verfahren und eine Vorrichtung der eingangs genannten Art zu schaffen, das bzw. die biotechnologisch arbeiten. Das erfindungsgemäße Verfahren ist, diese Aufgabe lösend, dadurch gekennzeichnet, daß der zerkleinerte Altgummi in einer Bakteriensuspension von chemolithotrophen Mikroorganismen unter Luftzufuhr gehalten wird, bis der Schwefel als elementarer Schwefel und/oder Schwefelsäure partiell oder vollständig vom verbleibenden replastizierten Kautschukregenerat abgetrennt ist.

Bei dem erfindungsgemäßen Verfahren wird der Schwefel des Altgummis partiell oder vollständig abgespalten und wird der abgespaltene Schwefel gewonnen und einer Wiederverwertung zugeführt. Auch das Kautschukregenerat wird einer Wiederverwertung zugeführt und ist auch dann wiederverwertbar, wenn der Schwefel nicht vollständig abgespalten ist. Chemolithotrophe Bakterien, z.B. der Gattung Thiobacillus, sind bei Anwesenheit von Luft (Sauerstoff und Kohlenstoff) in der Lage, die Sulfidbrücken des Gummis zu spalten und damit das Gummimaterial zu replastizieren, wodurch eine Weiterverarbeitung der vom Schwefel abgespaltenen Kohlenstoffketten und eine Wiederverwendung der von den Bakterien freigesetzten Schwefelverbindungen ermöglicht wird. Dabei werden von den Bakterien in Abhängigkeit von zugeführter Luft elementarer Schwefel und/oder Schwefelsäure durch Oxidation produziert. Das erfindungsgemäße Verfahren arbeitet mit verringertem Aufwand, ohne Chemikalien, rascher und mit besserer Ausbeute. Mit dem Schwefel werden auch Zinkoxid und andere Metalloxide abgespalten und in die Suspension überführt. Andere Zusatzstoffe des Altgummis, z.B. Ruß, Bitumen oder Stearinsäure, bleiben im wesentlichen im Kautschukregenerat. Die durch Oxidation produzierten sauerstoffhaltigen Schwefelverbindungen werden aufkonzentriert oder neutralisiert.

Besonders zweckmäßig und vorteilhaft ist es, wenn die Schwefelabspaltung nur eine Oberflächenschicht der Altgummi-Teilchen erfaßt und der Kern der Teilchen im Altgummizustand bleibt. Die Dicke der Oberflächenschicht beträgt z.B. wenige µm. Dieses Regenerat läßt sich bestimmten Umständen verbessert angepaßt weiterverarbeiten.

Besonders zweckmäßig und vorteilhaft ist es, wenn die chemolithotrophen Bakterien Thiobacillus ferrooxidans, Thiobacillus thiooxidans und/oder Thiobacillus thioparis sind. Diese Bakterienarten der Gattung Thiobacillus sind einfach zu handhaben und am Wirken zu halten.

Besonders zweckmäßig und vorteilhaft ist es auch, wenn bei Verwendung von Thiobacillus ferrooxidans und Thiobacillus thiooxidans der pH-Wert der Bakteriensuspension bei 1 bis 4, vorzugsweise bei 1,5 bis 2,5, gehalten wird. Bei Verwendung von Thiobacillus thioparis sind der pH-Wert bei 4 bis 7, vorzugsweise bis 5,5 bis 7, gehalten. In diesen pH-Wertbereichen sind die chemolithotrophen Mikroorganismen in verbesserter Weise schwefelabspaltend wirksam. Es wird der pH-Wert mittels einer Meßeinrichtung kontinuierlich gemessen. Die pH-Werteinstellung erfolgt z.B. durch geregelte Zugabe von Nährlösung und/ oder einer Chemikalie, beispielsweise NaOH.

Der abgespaltene Schwefel geht in die Bakteriensuspension über. Besonders zweckmäßig und vorteilhaft ist es daher, wenn der elementare Schwefel und/oder die Schwefelsäure zusammen mit Bakteriensuspension abgetrennt wird und dann von der Bakteriensuspension separiert wird. Dies ist eine einfache Art, den abgespaltenen Schwefel selbst darzustellen.

Die Vorrichtung zur Durchführung des erfindungsgemäßen Verfahrens läßt sich z.B. als Haldenreaktor gestalten, bei dem die Bakteriensuspension von oben über den zerkleinerten Altgummi, der in einem flachen Becken liegt, gesprüht wird, wobei der Altgummi der Luft ausgesetzt ist. Eine bevorzugte Ausführungsform der Erfindung ist dadurch gekennzeichnet, daß ein Becken zur Aufnahme der Bakteriensuspension bis zu einem Oberflächenniveau vorgesehen ist und dem Becken eine rotierend gelagerte Siebtrommel zugeordnet ist, die das Oberflächenniveau teilweise unterragt und teilweise überragt.

Auch hier ist keine gesonderte Luftzufuhr vorgesehen und ist der Sauerstoff der Luft genutzt. Diese Vorrichtung arbeitet kontrollierter und schneller als der Haldenreaktor. Durch die rotierende Trommel werden die Altgummi-Teilchen bewegt, gemischt und wiederholt der Luft ausgesetzt sowie in die Bakteriensuspension getaucht.

Eine weitere bevorzugte Ausführungsform der Erfindung ist dadurch gekennzeichnet, daß eine rotierend gelagerte geschlossene Trommel, die mit sich radial erstreckenden unterteilenden Wandungen versehen ist, zur Aufnahme des zerkleinerten Altgummis und der Bakteriensuspension vorgesehen ist. Während die Siebtrommel in der Regel bei Korngrößen über 80 µm eingesetzt wird, wird die vorliegende geschlossene Trommel in der Regel bei Korngrößen unter 80 µm eingesetzt. In der Regel und in Anpassung an die chemolithotrophen Mikroorganismen weist der zerkleinerte Altgummi eine Teilchengröße 50 µm bis 1.000 µm vorzugsweise 50 µm bis 350 µm, auf.

Die Zerkleinerung des Altgummis zu Granulat erfolgt nach bekannten Verfahren, wie z.B. durch Abkühlung mit Hilfe von verflüssigtem Stickstoff (DE 2803859; DE 2145728) oder verfestigtem Kohlendioxid (DE 2638387) und anschließender Zerkleinerung in üblichen Mahleinrichtungen, wie z.B. Hammermühlen, bis zu Korngrößen von 1 bis 15 mm, vorzugsweise von 5-7mm. Eine Aufarbeitung des bei der Runderneuerung von Altreifen anfallenden Gummimehls ist auch ohne vorherige Zerkleinerung möglich.

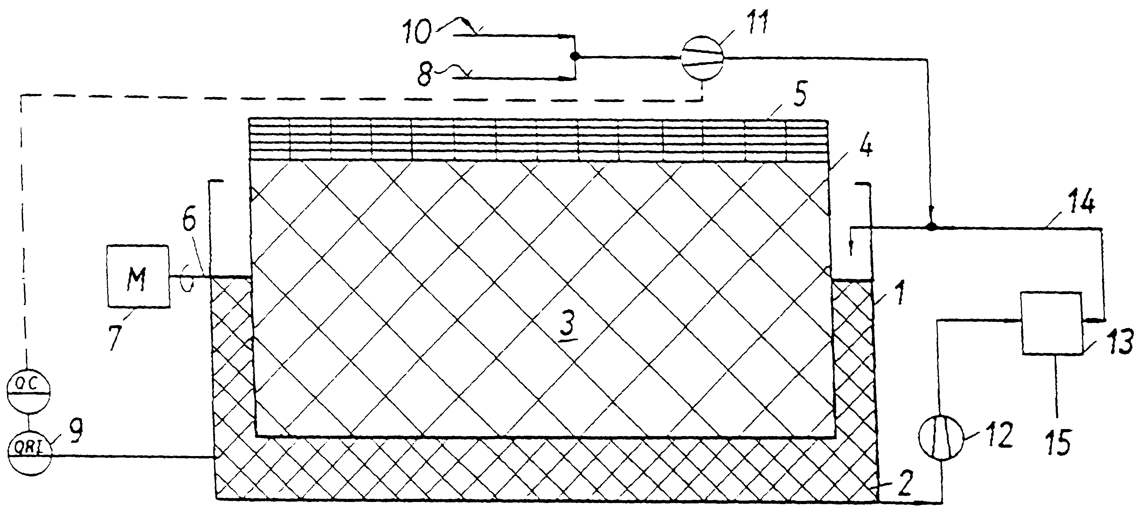

In der Zeichnung ist schematisch und teilweise im Schnitt eine bevorzugte Vorrichtung zur Aufarbeitung von Altgummi dargestellt.

Es wird Granulat von Altgummi nach der Zerkleinerung einem wannenartigen Behälter 1 zugeführt, in dem sich eine Bakteriensuspension 2 bei gleichzeitiger Anwesenheit von Sauerstoff befindet. Die Vorrichtung ist wahlweise so gestaltet, daß entweder ein chargenweiser oder aber ein quasikontinuierlicher bzw. ein kontinuierlicher Aus- und Eintrag des frischen, das heißt des aufzuarbeitenden Gummimaterials und des aufgearbeteten, schwefelarmen Gummigranulats möglich ist. Bei der vorliegenden Vorrichtung ist vorgesehen, daß das Gummigranulat in einen oder mehrere Trommelkörbe 4, bestehend aus Maschendraht nichtrostenden Stahls, überführt und dem oder den Trommelkörben 4 am oberen Teil des Behälters 1, z.B. mittels einer Hebevorrichtung, aufgegeben wird. Der oder die Trommelkörbe 4 sind mit einer Be- bzw. Entladeklappe 5 ausgestattet und auf einer Welle 6 derartig gelagert und befestigt, daß ein ständiges und gleichmäßiges Rotieren der Trommelkörbe 4 durch einen mit der Welle 6 gekoppelten Motorantrieb 7 gewährleistet ist. Nach der Aufarbeitung des Gummimaterials werden die Trommelkörbe 4 dem Behälter 1 wieder entnommen und das schwefelarme Gummimaterial bzw. Kautschukregenerat einer gesonderten Weiterverarbeitung zugeführt. Durch die ständige Rotation der mit Gummigranulat 3 gefüllten Trommelkörbe 4, welche zu etwa 30-40 Mengenanteilen, vorzugsweise 30-35 Mengenanteilen des eingefüllten Gummigranulats in die Bakteriensuspension eintauchen, wird das Gummigranulat 4 kontinuierlich von der sich im Behälter 1 befindenden Bakteriensuspension 2 umspült.

Der Behälter 1 ist z.B. als Becken gestaltet, bezüglich dessen Flüssigkeitsoberfläche die Trommelkörbe 4 rotieren, so daß ein ausreichender Kontakt zwischen der Bakteriensuspension 2 und dem Gummigranulat 3 gewährleistet ist. Weiterhin wird durch das Rotieren der Trommelkörbe 4 an der Flüssigkeitsoberfläche eine Vergrößerung der Stoffübergangsfläche zwischen Flüssigkeit bzw. Suspension 2 und Luft erreicht, so daß auf eine gesonderte Sauerstoffversorgung zur Begasung der Suspension verzichtet werden kann.

Der Bakteriensuspension 2 wird ständig oder in bestimmten Zeitintervallen, beispielsweise täglich, eine definierte Menge an Nährlösung und Spurenelementen über eine Zuleitung 8 zugegeben. Der pH-Wert der Bakteriensuspension 2 wird ständig mit einer kontinuierlichen pH-Meßeinrichtung 9 gemessen und auf die für die Aufarbeitung jeweils optimalen pH-Werte eingestellt. Die Regelung des pH-Wertes kann durch Zugabe der notwendigen Nährlösung über die Zuleitung 8 und/oder durch Zugabe von Chemikalien über eine Zuleitung 10 und über eine nachfolgende Zuteileinrichtung 11 realisiert werden. Als weitere Ausführungsform des Bioreaktors sind z.B. Haldenraktoren verwendbar, so wie sie bei Laugungsprozessen von Armerzen angewendet werden, beschrieben z.B. in Torma, A.E. "Current standing heap, dump, in-situ leaching technology of copper"; Metall 38 (1984) S. 1044-1047.

Bei einem für die Bakterien limitierten Sauerstoffeintrag erfolgt vorrangig eine Oxidation des im Gummi gebundenen Schwefels zu elementarem Schwefel. Ein Teil der Bakteriensuspension 2 wird mit dem von den Bakterien produzierten Schwefel am Boden des Behälter 1 über eine Pumpe 12 abgezogen und einer Separationsstufe 13, beispielsweise einem Hydrozyklon oder einer Filterkerze, zur Abtrennung des Schwefels zugeführt. Nach Abtrennung des Schwefels in der Separationsstufe 13 wird die vom Schwefel befreite Suspension über eine Leitung 14 dem Behälter 1 erneut zugeführt.

## Patentansprüche

1. Verfahren zur Behandlung von Gummi,
bei dem der Gummi Schwefel enthält und zu Teilchen zerkleinert wird,
bei dem aus dem zerkleinerten Gummi unter Devulkanisierung Kautschukregenerat erzeugt wird,
bei dem der zerkleinerte Gummi zusammen mit chemolithotrophen Mikroorganismen gehalten wird, bis Schwefel als elementarer Schwefel und/oder Schwefelsäure vom verbleibenden replastizierten Kautschukregenerat abgetrennt ist, und
bei dem die chemolithotrophen Mikroorganismen in einer die Gummi-Teilchen aufnehmenden Bakteriensuspension vorliegen,
**dadurch gekennzeichnet,**
daß die Behandlung eine Aufarbeitung von Altgummi ist,
daß der Altgummi mit den Mikroorganismen unter Luftzufuhr gehalten wird und
daß die Altgummi-Teilchen in der Bakteriensuspension vorliegen,
bis die Schwefelabspaltung nur eine Oberflächenschicht der Altgummi-Teilchen erfaßt, wobei der Kern der Teilchen im Altgummizustand bleibt.

2. Verfahren nach Anspruch 1, **dadurch gekennzeichnet,** daß die chemolithotrophen Bakterien Thiobacillus ferrooxidans, Thiobacillus thiooxidans und/oder Thiobacillus thioparus sind.

3. Verfahren nach Anspruch 1 oder 2, **dadurch gekennzeichnet,** daß bei Verwendung von Thiobacillus ferrooxidans oder Thiobacillus thiooxidans der ph-Wert der Bakteriensuspension bei 1-4, vorzugsweise bei 1,5 - 2,5, gehalten wird.

4. Verfahren nach Anspruch 1 oder 2, **dadurch gekennzeichnet,** daß bei Verwendung von Thiobacillus thioparus der ph-Wert der Bakteriensuspension bei 4-7, vorzugsweise bei 5,5 - 7, gehalten wird.

5. Verfahren nach einem der vorhergehenden Ansprüche, **dadurch gekennzeichnet,** daß der zerkleinerte Altgummi eine Teilchengröße 50 µm bis 1.000 µm, vorzugsweise bis 350 µm aufweist.

## Claims

1. A method of processing rubber,
wherein the rubber contains sulphur and is comminuted to form particles,
wherein reclaimed rubber is produced from the comminuted rubber during devulcanization,
wherein the comminuted rubber is held together with chemolithotrophic micro-organisms until sulphur is separated as elementary sulphur and/or sulphuric acid from the remaining replasticized reclaimed rubber, and
wherein the chemolithotrophic micro-organisms are present in a bacterial suspension which receives the rubber particles,
**characterized in that** the processing is a re-conditioning of old rubber,
the old rubber is kept with the micro-organisms while air is supplied, and
the old-rubber particles are present in the bacterial suspension,
until the splitting of sulphur affects only a surface layer of the old-rubber particles, wherein the core of the particles remains in the old-rubber state.

2. A method according to Claim 1, **characterized in that** the chemolithotrophic bacteria are *Thiobacillus ferrooxidans, Thiobacillus thiooxidans* and/or *Thiobacillus thioparus.*

3. A method according to Claim 1 or 2, **characterized in that** when *Thiobacillus ferrooxidans* or *Thiobacillus thiooxidans* is used the pH value of the bacterial suspension is held at from 1 to 4, preferably at 1·5 to 2·5.

4. A method according to Claim 1 or 2, **characterized in that** when *Thiobacillus thioparus* is used the pH value of the bacterial suspension is held at from 4 to 7, preferably at 5·5 to 7.

5. A method according to one of the preceding Claims, **characterized in that** the comminuted old rubber has a particle size of from 50 µm to 1,000 µm, preferably up to 350 µm.

## Revendications

1. Procédé de traitement du caoutchouc dans lequel le caoutchouc contient du soufre et est broyé en particules, dans lequel du caoutchouc régénéré est produit à partir du caoutchouc broyé sous dévulcanisation, dans lequel le caoutchouc broyé est maintenu avec des micro-organismes chimiolithotrophes jusqu'à ce que le soufre soit séparé sous forme de soufre élémentaire et/ou d'acide sulfurique du caoutchouc régénéré replastifié restant, et dans lequel les micro-organismes chimiolithotrophes sont présents dans une suspension bactérienne recevant les particules de caoutchouc, caractérisé en ce que le traitement est un retraitement de caoutchouc usagé, en ce que le caoutchouc usagé est maintenu avec les micro-organismes sous apport d'air et en ce que les particules de caoutchouc usagé sont situées dans la suspension bactérienne jusqu'à ce que la séparation du soufre ne concerne qu'une couche superficielle des particules de caoutchouc usagé, le noyau des particules restant à l'état de caoutchouc usagé.

2. Procédé selon la revendication 1, caractérisé en ce que les bactéries chimiolithotrophes sont Thiobacillus ferrooxidans, Thiobacillus thiooxidans et/ou Thiobacillus thioparus.

3. Procédé selon la revendication 1 ou 2, caractérisé en ce que, lorsqu'on utilise Thiobacillus ferrooxidans ou Thiobacillus thiooxidans, le pH de la suspension bactérienne est maintenu entre 1 et 4, de préférence entre 1,5 et 2,5.

4. Procédé selon la revendication 1 ou 2, caractérisé en ce que, lorsqu'on utilise Thiobacillus thioparus, le pH de la suspension bactérienne est maintenu entre 4 et 7, de préférence entre 5,5 et 7.

5. Procédé salon l'une des revendications précédentes, caractérisé en ce que le caoutchouc usagé broyé présente une taille de particule de 50 µm à 1 000 µm, de préférence de jusqu'à 350 µm.
